(19) Europäisches Patentamt
European Patent Office
Office européen des brevets

(11) Publication number: 0 132 542 B1

# (12) EUROPEAN PATENT SPECIFICATION

(45) Date of publication of patent specification: 08.10.86

(51) Int. Cl.$^4$: C 07 C 17/08

(21) Application number: 84106219.3

(22) Date of filing: 30.05.84

## (54) Hydrohalogenation of conjugated dienes in the presence of organic amines.

(30) Priority: 20.07.83 US 515563

(43) Date of publication of application:
13.02.85 Bulletin 85/07

(45) Publication of the grant of the patent:
08.10.86 Bulletin 86/41

(84) Designated Contracting States:
BE CH DE FR GB LI NL

(56) References cited:
DE-A-1 768 544
DE-A-2 538 636
US-A-2 882 323

(73) Proprietor: Union Camp Corporation
1600 Valley Road
Wayne New Jersey 07470 (US)

(72) Inventor: McElligott, Lois T.
1158 Wheatsheaf Lane
Abington, PA 19001 (US)

(74) Representative: von Füner, Alexander, Dr. et al
Patentanwälte v. Füner, Ebbinghaus, Finck
Mariahilfplatz 2 & 3
D-8000 München 90 (DE)

EP 0 132 542 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

## Background of the Invention

Field of the Invention

The invention relates to processes for the hydrohalogenation of conjugated diene hydrocarbons.

Brief Description of the Prior Art

The literature is replete with descriptions of processes for the hydrohalogenation of conjugated dienes. Representative of such descriptions are those found in the U.S. Patents, 2,882,323 and 3,016,408 and in British Patent 896,262; see also German Offenlegungsschrift 1,768,544. In the latter patent, hydrohalogenation of butadiene is conducted in the presence of aqueous mixtures including organic amines. The reaction mixtures are corrosive and in some reactions would tend to promote solvolysis. The German Auslegeschrift 1,253,264 describes the hydrochlorination of butadiene in a gas phase reaction. The present invention is an improvement over the prior art processes in that it may be carried out in liquid phases of the reactants, at low temperatures and with minimal solvolysis of products. Improved yields are obtained of the desired products.

The present invention is particularly advantageous when used to hydrochlorinate myrcene. Myrcene is a conjugated diene of the formula:-

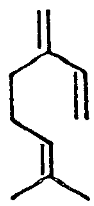

(I)

When hydrochlorinated in the absence of any catalyst, the major product is myrcenyl chloride. However, the commercially valuable products of myrcene hydrochlorination are the associated co-products, namely, geranyl chloride and neryl chloride. Hydrochlorination in the presence of a copper catalyst shifts the reaction in favor of the desired co-products. It has been postulated that the hydrochlorination of myrcene in the presence of a copper catalyst proceeds according to the reaction scheme:-

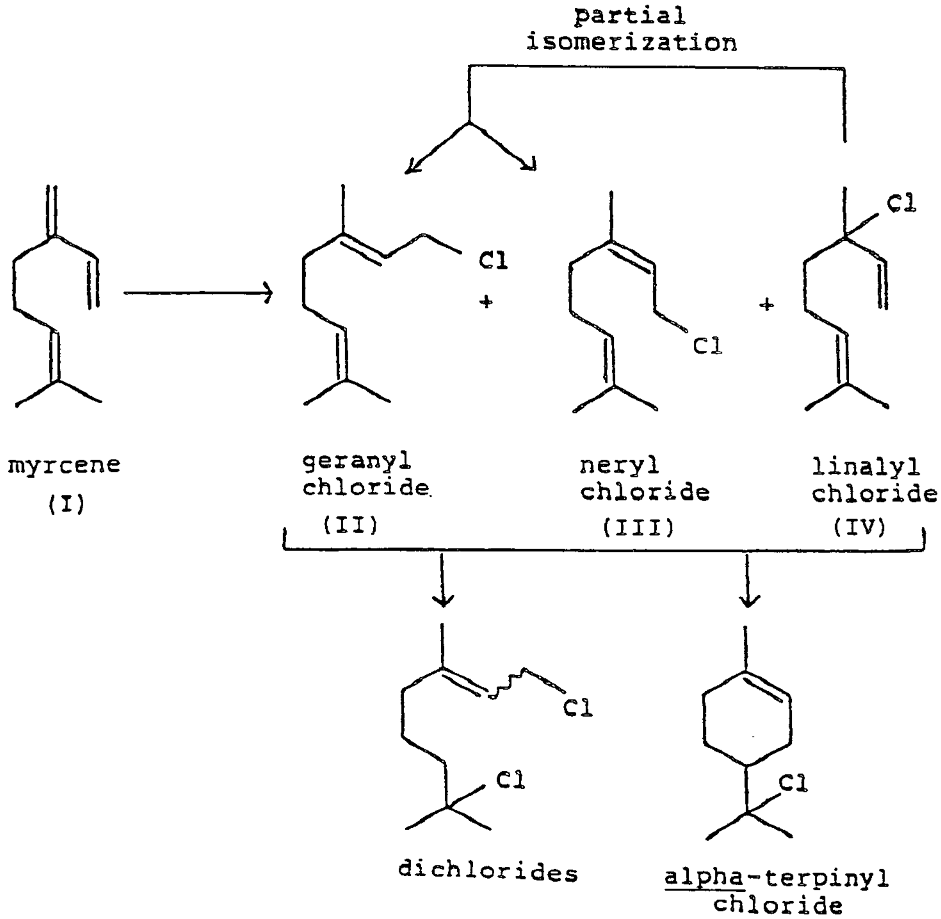


myrcene (I)

geranyl chloride (II)

neryl chloride (III)

linalyl chloride (IV)

dichlorides (V)

alpha-terpinyl chloride (VI)

Cyclization may also undesirably occur to form the *alpha*-terpinyl chlorides.

When the copper catalyst employed is in the form of cupric chloride ($CuCl_2$) the products generally include substantial proportion so linalyl chloride and lesser proportions than the desired geranyl and neryl chlorides. When the copper catalyst is in the form of cuprous chloride, the linalyl chloride product is lessened due, apparently, to partial isomerization to the desired geranyl and neryl chlorides.

From the above proposed reaction scheme, it will be appreciated that any process for hydrochlorination of myrcene, to be commercially feasible, must result in a favorable yield of the desired geranyl (II) and neryl (III) monochlorides and minimal formation of linalyl (IV) and *alpha*-terpinyl (VI) monochlorides. It was previously appreciated that the relative proportions of monochlorides (II), (III) and (IV) in the hydrochlorination product reaction mixture could be controlled to some degree by selection of the reaction temperature, gas flowrate and catalyst concentration.

We have now found that when the prior art hydrohalogenation of a conjugated diene, such as the hydrochlorination of isoprene and in particular the hydrochlorination of myrcene, is carried out in the presence of an organic amine, then the isomerization of the allylic chloride products during the hydrohalogenation reaction is shifted to favor formation of the less-substituted allylic chloride, being geranyl chloride (II) and neryl chloride (III) in the above example.

The advantages associated with the improved process of the invention include improved overall yield of the more desirable chloride, for example prenyl chloride from isoprene and neryl and geranyl chlorides from myrcene, and for the latter case, a greater selectivity of the more important geranyl isomer.

## Summary of the Invention

The invention comprises, in a method for the hydro-halogenation of a conjugated diene which comprises, hydrohalogenating the diene under anhydrous, liquid phase conditions in the presence of a catalytic proportion of a hydrohalogenation catalyst, the improvement which comprises; carrying out the hydrohalogenation in the presence of an organic amine.

The improved process of the invention may be carried out in a batch or a continuous manner.

It is preferred that the amine contains at least 6 carbon atoms whereby especially preferred are trihexylamine, a mixture of tri(alkyl)amines where the alkyl portion comprises a chain of from eight to ten carbon atoms, trioctylamine, tri(tridecyl)amine, distearylmethylamine, dihydrogenated tallow methylamine, tridecylamine and tri(docecyl)amine.

Preferred dienes are myrcene, isoprene and ocimene whereby halogen should be chlorine and the catalyst is a copper catalyst.

An improved process is carried out at a temperature of from about −30°C to 50°C.

As catalyst cuprous chloride is preferred.

The catalytic proportion of copper catalyst should especially be within a weight range of 0.01 to 10.0 percent of the dry diene.

It is preferred to carry out the process for a period of from 3 to 15 hours.

Finally the molar ratio of amine to copper catalyst should preferably be within the range of from 0.1 to 5.0.

## Brief Description of the Drawing

The drawing is a schematic representation of a preferred embodiment method of the invention.

## Detailed Description of the Preferred Embodiments of the Invention

The process of the invention may be employed for the hydrohalogenation of any conjugated diene. Representative of such dienes are myrcene, isoprene, 2-methylpiperylene, *beta*-phellandrene, 2-ethylbutadiene, ocimene, alloocimene, 1-phenylbutadiene, and the like.

The method of the invention is particularly advantageous for the hydrohalogenation of myrcene to obtain the geranyl and neryl halides which are intermediates for the manufacture of commercially valuable geraniol and nerol. When applied to myrcene, the improved method of the invention will increase the overall yield of the geranyl and neryl halides over the prior art processes and will improve selectivity of the ratio of geranyl isomer to the neryl isomer. Commercially available myrcene made by pyrolysis of *beta*-pinene, purified forms of myrcene, and myrcene isolated from natural materials may be provided as the starting material in the preferred process of the invention.

The accompanying drawing is a schematic representation of a preferred embodiment method of the invention for the hydrochlorination of myrcene. As shown in the Figure the provided myrcene initially held in tank 10 may be first dried in a conventional salt bed dryer 20 to remove water, as necessary. The dried myrcene is then preferably cooled to a temperature in the range of from about −30°C. to about 30°C.; most preferably circa 10°C. in a cooling unit 30. Alternatively, the myrcene may be cooled first and then processed through a salt bed dryer 20 to remove water. Cooling the starting myrcene prior to drying in the salt bed dryer 20 is somewhat advantageous in that pre-cooling increases drying efficiency in the salt bed dryer 20.

As shown in the Figure the dried and cooled myrcene starting material may be passed into a hydrohalogenation apparatus which comprises in the preferred embodiment a stirred tank reactor 40. In reactor 40, hydrohalogenation of the introduced myrcene is carried out in the presence of a catalytic proportion of a hydrohalogenation catalyst, at a temperature within the range of from about −30°C. to

about 50°C.; preferably at a temperature within the range of from −10°C. to 25°C., most preferably about 10°C. Hydrohalogenation may be effected, for example, by reaction of the myrcene with a hydrogen halide like hydrogen chloride or hydrogen bromide, in substantially anhydrous form and under substantially anhydrous conditions, i.e. having less than about 5% water present in the reaction mixture. As shown in the Figure, the preferred hydrohalogenation is with gaseous hydrogen chloride which is introduced as a gas possibly generated in a vaporizer, and then metered into the reactor 40, via appropriate conduits. Advantageously, the hydrogen chloride is metered into the reactor 40 at a rate of from about 2.0 to about 300 gms/hour/mole of myrcene present in the reactor 40. Preferably, the rate is from about 4.0 to 8.0 gms/hour/mole of myrcene.

The organic amine is introduced into the reactor 40 from storage vessel 50. Myrcene, amine, hydrogen chloride and copper catalyst are introduced into the reactor 40 sequentially. Preferably, the reactor 40 after purging with an inert gas such as nitrogen is first charged with the cool myrcene, the copper catalyst, and the organic amine. While cooling and stirring, the hydrogen chloride is added incrementally to the charge.

A wide variety of catalysts for hydrohalogenation of myrcene are well known and include, for example, any copper compound having a valency of 2 or less, including metallic copper. Any copper compound convertible to the halide such as the bromide, iodide or chloride under conditions of the reaction may also be used. Representative of copper catalysts advantageously employed are the chloride, bromide, carbonate, oxide, acetate, formate, sulfate, and like derivative cupric and cuprous compounds. Preferred as the hydrochlorination catalyst in the improved process of the invention is cuprous chloride. Catalytic proportions of the anhydrous hydrohalogenation catalyst are generally within the weight range of from about 0.01 to 10 percent of the dry myrcene, preferably about 0.5 percent.

Organic amines are generally well-known in the art as is their preparation and include primary, secondary and tertiary amines. Representative of organic amines which may be used in the process of the invention are those of the formula:-

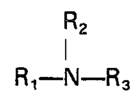

(VII)

wherein $R_1$, $R_2$ and $R_3$ are each independently selected from the group consisting of hydrogen and hydrocarbyl, at least one of $R_1$, $R_2$ and $R_3$ being hydrocarbyl. Also representative of organic amines which may be used in the method of the invention are those of the formula (VII) given above, wherein $R_1$ and $R_2$ are taken together to represent a divalent moiety attached to the atom N, and which is selected from the group consisting of alkenylene and hydrocarbyl-substituted alkenylene having 5 to 10 carbon atoms, inclusive, in the ring thereof; or $R_1$ and $R_2$ may be taken together with the atom of N to which they are attached to represent a divalent or monovalent moiety selected from the groups consisting of those having the formula:-

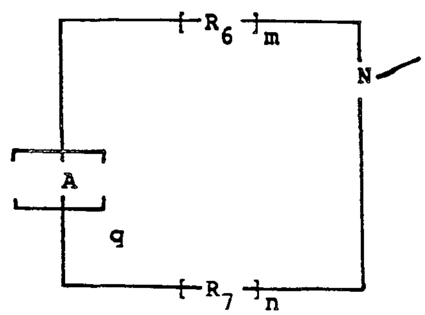

wherein A represents nitrogen, oxygen, sulfur, phosphorus and the like; and $R_6$ and $R_7$ are each selected from alkenylene and hydrocarbyl-substituted alkenylene of 1 to 25 carbon atoms, inclusive, m, n and q are each integers of 0 to 1 and the sum of m + n is 1 or 2.

The term "hydrocarbyl" as used herein means the monovalent moiety obtained upon removal of a hydrogen atom from a parent hydrocarbon. Representative of hydrocarbyl are alkyl of 1 to 25 carbon atoms, inclusive, such as methyl, ethyl, propyl, butyl, pentyl, hexyl, hepty, octyl, nonyl, undecyl, decyl, dodecyl, octadecyl, nonodecyl, eicosyl, heneicosyl, docosyl, tricosyl, tetracosyl, pentacosyl and the isomeric forms thereof; aryl of 6 to 25 carbon atoms, inclusive, such as phenyl, tolyl, xylyl, naphthyl, biphenyl, tetraphenyl and the like; aralkyl of 7 to 25 carbon atoms, inclusive, such as benzyl, phenethyl, phenpropyl, phenbutyl, phenhexyl, naphthoctyl and the like; cycloalkyl of 3 to 8 carbon atoms, inclusive, such as cyclopropyl, cyclobutyl, cyclopentyl, cyclohexyl, cycloheptyl, cyclooctyl and the like; alkenyl of 2 to 25 carbon atoms, inclusive, such as vinyl, allyl, butenyl, pentenyl, hexenyl, octenyl, nonenyl, decenyl, undececyl, dodecenyl, tridecényl, pentadecenyl, octadecenyl, pentacosynyl and isomeric forms thereof.

The term "alkenylene" means the divalent moiety obtained on removal of two hydrogen atoms, each from a non-adjacent carbon atom of a parent hydrocarbon and includes alkenylene of 3 to 10 carbon atoms,

inclusive, such as 1,3-propenylene, 1,4-butenylene, 1,5-pentenylene, 1,8-octenylene, 1,10-decenylene and the like.

The terms "substituted hydrocarbyl" and "substituted alkenylene" as used herein mean the hydrocarbyl or alkenylene moiety as previously defined wherein one or more hydrogen atoms have been replaced with an inert group, i.e. a chemical group which does not adversely affect the desired function of the organic amine of formula (VII). Representative of such groups are aminophosphino-hydrocarbyl, quaternary nitrogen (ammonium), quaternary phosphorus (phosphonium), hydroxyl-, alkoxy, mercapto-, alkyl, halo-, phosphate, phosphite, carboxylate groups and the like.

Organic amine compounds of the formula (VII) given above are generally well-known as are methods of their preparation. Representative of such organic amine compounds are:

Methylamine
Ethylamine
Dimethylamine
n-Propylamine
Isopropylamine
Trimethylamine
n-Butylamine
s-Butylamine
Isobutylamine
t-Butylamine
Methylisopropylamine
n-Amylamine
2-Aminopentane
3-Aminopentane
Isoamylamine
t-Amylamine
Methyl-n-butylamine
Ethyl-n-propylamine
N,N-Diethylmethylamine
n-Hexylamine
Cyclohexylamine
2-Methyl-4-aminopentane
2,2-Dimethyl-3-aminobutane
Ethyl-n-butylamine
Dimethyl-n-butylamine
Triethylamine
n-Heptylamine
2-Aminoheptane
n-Propyl-n-butylamine
Isopropyl-n-butylamine
Diethylisopropylamine
Ethyl-n-hexylamine
Di-n-butylamine
Di-n-hexylamine
3-Methyl-1-aminocyclohexane
4-Methyl-1-aminocyclohexane
N-Cyclohexylethylamine
trans-2-Ethylcyclohexylamine
N-Ethylcyclohexylamine
1-Cyclohexyl-2-aminopropane
N-Methyl-cyclohexylethylamine
9-Aminodecalin
Dicyclohexylamine
a-Phenylethylamine
Phenylethylamine
o-Methylbenzylamine
p-Methylbenzylamine
p-Ethylaniline
3-Amino-1,2-dimethylbenzene
1,3-Dimethyl-5-aminobenzene
N-Methylbenzylamine
N,N-Dimethylaniline
Piperazine
N,N'-Dimethylpiperazine
N-Methylmorpholine

Pyridine
Aniline

and the like.

Preferred compounds of the formula (VII) are those wherein the carbon atom content in $R_1$ plus $R_2$ plus $R_3$ totals at least 6.

It will be appreciated that under specific conditions of operating the process of the invention, certain of the above described compounds of the formula (VII) given above have advantages over other compounds of the same general formula. Selection of a particular compound (VII) for use under specific process conditions, for optimum yields may be made by trial and error technique.

The organic amine is used in a proportion to isomerize, during the hydrohalogenation reaction, at least some of the more-substituted allylic chloride produced in the method of the invention. Such a proportion is generally within the range of from about 0.01 to 10 percent by weight of the diene charge, preferably 0.2 to 2.5 percent. Optimum proportions will depend to some extent upon the amine selected and may be determined by trial and error technique.

The controlling reaction rate in the hydrohalogenation process of the invention is the isomerization of the more-substituted halide to the desired less-substituted halide. This is controlled by residence time in the hydro-halogenation reaction zone. We have found that in hydrochlorination of myrcene, the preferred minimum total residence time is within the range of from 3 to 15 hours, and most preferably 5 to 8 hours under the above described operating temperatures. The presence of linalyl chloride in the reaction mixture may be monitored by conventional analytical techniques. Longer residence times in the hydrochlorination reactor may cause a yield loss due to conversion of the monochlorides to *alpha*-terpinyl chloride. Shorter residence times may not be sufficient to isomerize the linalyl chloride to the desired geranyl/neryl chlorides.

When it has been determined that hydrohalogenation has occurred to a maximum desired point, the hydrohalogenation product mixture is passed from the hydrohalogenation apparatus. The desired hydrohalogenated diene may be separated from the reaction mixture employing conventional and known techniques including washing, decantation, distillation and like techniques.

The following examples describe the manner and process of making and using the invention and set forth the best mode contemplated by the inventors of carrying out the invention but are not to be construed as limiting. All parts given are by weight unless otherwise indicated.

Examples 1—7

To a 1 liter reaction vessel was charged 300.0 g myrcene (72 wt%), 1.0 g cuprous chloride and an amine as described in Table 1. The mixture was purged with nitrogen and cooled to 0°C. Hydrogen chloride gas was added at a rate of 7—8g/hour while maintaining the temperature at 10°C. At the end of the reaction (7 hours), monitored by infrared spectroscopy to a 1% myrcene level, the reaction product was neutralized with sodium carbonate and aqueous sodium hydroxide. The product was analyzed by gas chromatography. The results are shown in Table 1. Linalyl, neryl and geranyl chlorides are abbreviated LCl, NCl, and GCl respectively.

TABLE 1

| Example No. | Amine Used | Molar Yield of Products LCI | NCl+GCl | GCl:NCl Ratio |
|---|---|---|---|---|
| 1 | None | 18 | 69 | 1.26 |
| 2 | Trihexylamine, 2.7g | 13 | 78 | 1.59 |
| 3 | Trioctylamine, 3.5g | 12 | 77 | 1.60 |
| 4 | Adogen 364*, 3.9g | 10 | 81 | 1.54 |
| 5 | Tri (tridecyl)-amine, 5.7g | 14 | 78 | 1.82 |
| 6 | Dihydrogenated tallow methylamine, 5.1g | 9 | 81 | 1.76 |
| 7 | Distearyl methylamine, 5.5g | 15 | 71 | 1.41 |

* A mixture of trialkylamines where the alkyl chains comprise 59% $C_8$; 39% $C_{10}$; 1% $C_{12}$ and 1% $C_6$; Sherex Chemical Company.

Example 8

To a chilled 50 ml reaction vessel was charged 10.0g isoprene, 0.1g cuprous chloride and Adogen 364, supra., in an amount indicated in Table 2. The mixture was cooled to 0°C. Hydrogen chloride gas was added at a rate of 1.5—2.0 g/hour while maintaining the temperature at 0°C. At the end of the reaction (3 hours), monitored by weight, the reaction product was neutralized and analyzed by nuclear magnetic resonance spectroscopy to determine the amounts of 3-chloro-3-methyl-1-butene and 1-chloro-3-methyl-2-butene (abbreviated 3,3,1-B and 1,3,2-B respectively). The result of this analysis is shown in Table 2, below.

TABLE 2

| Amount Adogen 364 Used | Composition of Product (%) 3,3,1-B | 1,3,1-B |
|---|---|---|
| None | 28 | 72 |
| 0.35g | 12 | 88 |

**Claims**

1. In a method for the hydrohalogenation of a conjugated diene which comprises hydrohalogenating the diene under anhydrous, liquid phase conditions in the presence of a catalytic proportion of a hydrohalogenation catalyst, the improvement which comprises; carrying out the hydrohalogenation in the presence of an organic amine.

2. The method of claim 1 wherein the amine contains at least 6 carbon atoms.

3. The improved method of claim 2 wherein the organic amine is trihexylamine.

4. The improved method of claim 1 wherein the organic amine is a mixture of tri(alkyl)amines where the alkyl portion comprises a chain of from eight to ten carbon atomes.

5. The improved method of claim 2 or 4 wherein the organic amine is trioctylamine.

6. The improved method of claim 2 wherein the organic amine is tri(tridecyl)amine.

7. The improved method of claim 2 wherein the organic amine is distearylmethylamine.

8. The improved method of claim 2 wherein the organic amine is dihydrogenated tallow methylamine.

9. The improved method of claim 2 wherein the organic amine is tridecylamine.

10. The improved method of claim 2 wherein the organic amine is tri(dodecyl)amine.

11. The improved method of one of the claims 1—10 wherein the diene is myrcene, the halogen is chlorine and the catalyst is a copper catalyst.

12. The improved method of one of the claims 1—10 wherein the diene is isoprene, the halogen is chlorine and the catalyst is a copper catalyst.

13. The improved method of one of the claims 1—10 wherein the diene is ocimene, the halogen is chlorine and the catalyst is a copper catalyst.

14. The improved process of one of the claims 1—13 wherein the temperature of reaction is from about −30°C to 50°C.

15. The process of one of the claims 1—14 wherein the catalyst is cuprous chloride.

16. The process of one of the claims 1—15 wherein the catalytic proportion of copper catalyst is within the weight range of 0.01 to 10.0 percent of the dry diene.

17. The process of one of the claims 1—16 wherein the hydrohalogenation is carried out for a period of from 3 to 15 hours.

18. The process of one of the claims 1—17 wherein the molar ratio of amine to copper catalyst is within the range of from 0.1 to 5.0.

**Revendications**

1. Dans un procédé d'hydrohalogénation d'un diène conjugué qui comprend l'hydrohalogénation du diène en phase liquide anhydre en présence d'une proportion catalytique d'un catalyseur d'hydrohalogénation, l'amélioration qui consiste à mettre en oeuvre l'hydrohalogénation en présence d'une amine organique.

2. Le procédé selon la revendication 1, dans lequel l'amine contient au moins 6 atomes de carbone.

3. Le procédé amélioré selon la revendication 2, dans lequel l'amine organique est la trihexylamine.

4. Le procédé amélioré selon la revendication 1, dans lequel l'amine organique est un mélange de tri(alkyl)amines où la partie alkylique comprend une chaîne formée de 8 à 10 atomes de carbone.

5. Le procédé amélioré selon la revendication 2 ou 4, dans lequel l'amine organique est la trioctylamine.

6. Le procédé amélioré selon la revendication 2, dans lequel l'amine organique est la tri(tridécyl)amine.

7. Le procédé amélioré selon la revendication 2, dans lequel l'amine organique est la distéarylméthylamine.

8. Le procédé amélioré selon la revendication 2, dans lequel l'amine organique est la méthylamine de suif déshydrogéné.

9. Le procédé amélioré selon la revendication 2, dans lequel l'amine organique est la tridécylamine.

10. Le procédé amélioré selon la revendication 2, dans lequel l'amine organique est la tri(dodécyl)amine.

11. Le procédé amélioré selon l'une quelconque des revendications 1 à 10, dans lequel le diène est le myrcéne, l'halogène est le chlore et le catalyseur à base de cuivre.

12. Le procédé amélioré selon l'une quelconque des revendications 1 à 10, dans lequel le diéne est l'isoprène, l'halogène est le chlore et le catalyseur est un catalyseur à base de cuivre.

13. Le procédé amélioré selon l'une quelconque des revendications 1 à 10, dans lequel le diène est l'ocimène, l'halogène est le chlore et le catalyseur est un catalyseur à base de cuivre.

14. Le procédé amélioré selon l'une quelconque des revendications 1 à 13, dans lequel la température de réaction est comprise entre −30°C et 50°C.

15. Le procédé selon l'une quelconque des revendications 1 à 14, dans lequel le catalyseur est le chlorure cuivreux.

16. Le procédé selon l'une quelconque des revendications 1 à 15, dans lequel la proportion catalytique de catalyseur à base de cuivre est comprise entre les proportions pondérales de 0,01 et de 10,0% par rapport au diène sec.

17. Le procédé selon l'une quelconque des revendications 1 à 16, dans lequel l'hydrohalogénation est mise en oeuvre pendant une période de 3 à 15 heures.

18. Le procédé selon l'une quelconque des revendications 1 à 17, dans lequel le rapport molaire amine/catalyseur à base de cuivre est compris entre 0,1 et 5,0.

**Patentansprüche**

1. Verfahren zur Hydrohalogenierung eines konjugierten Diens, worin das Dien unter wasserfreien Flüssigphasebedingungen in Gegenwart einer katalytischen Menge eines Hydrohalogenierungskatalysators hydrohalogeniert wird, wobei die Verbesserung darin besteht, daß die Hydrohalogenierung in Gegenwart eines organischen Amins durchgeführt wird.

2. Verfahren nach Anspruch 1, worin das Amin mindestens 6 Kohlenstoffatome enthält.

3. Verbessertes Verfahren nach Anspruch 2, worin das organische Amin Trihexylamin ist.

4. Verbessertes Verfahren nach Anspruch 1, bei dem das organische Amin ein Gemisch aus Tri(alkyl)aminen ist, worin der Alkylanteil eine Kette von 8 bis 10 Kohlenstoffatomen umfaßt.

5. Verbessertes Verfahren nach Anspruch 2 oder 4, worin das organische Amin Trioctylamin ist.

6. Verbessertes Verfahren nach Anspruch 2, worin das organische Amin Tri(tridecyl)amin ist.

7. Verbessertes Verfahren nach Anspruch 2, worin das organische Amin Distearylmethylamin ist.

8. Verbessertes Verfahren nach Anspruch 2, worin das organische Amin dihydriertes Talgmethylamin ist.

9. Verbessertes Verfahren nach Anspruch 2, worin das organische Amin Tridecylamin ist.

10. Verbessertes Verfahren nach Anspruch 2, worin das organische Amin Tri(dodecyl)amin ist.

11. Verbessertes Verfahren nach einem der Ansprüche 1 bis 10, worin das Dien Myrcen, das Halogen Chlor und der Katalysator ein Kupferkatalysator ist.

12. Verbessertes Verfahren nach einem der Ansprüche 1 bis 10, worin das Dien Isopren, das Halogen Chlor und der Katalysator ein Kupferkatalysator ist.

13. Verbessertes Verfahren nach einem der Ansprüche 1 bis 10, worin das Dien Ocimen, das Halogen Chlor und der Katalysator ein Kupferkatalysator ist.

14. Verbessertes Verfahren nach einem der Ansprüche 1 bis 13, worin die Reaktionstemperatur zwischen ungefähr −30°C und 50°C beträgt.

15. Verfahren nach einem der Ansprüche 1 bis 14, worin der Katalysator Cuprochlorid ist.

16. Verfahren nach einem der Ansprüche 1 bis 15, worin der katalytische Anteil des Kupferkatalysators innerhalb eines Gewichtsbereichs von 0,01 bis 10,0% des trockenen Diens liegt.

17. Verfahren nach einem der Ansprüche 1 bis 16, worin die Hydrohalogenierung während eines Zeitraums von 3 bis 15 Stunden durchgeführt wird.

18. Verfahren nach einem der Ansprüche 1 bis 17, worin das Molverhältnis des Amins zum Kupferkatalysator innerhalb eines Bereichs von 0,1 bis 5,0 liegt.

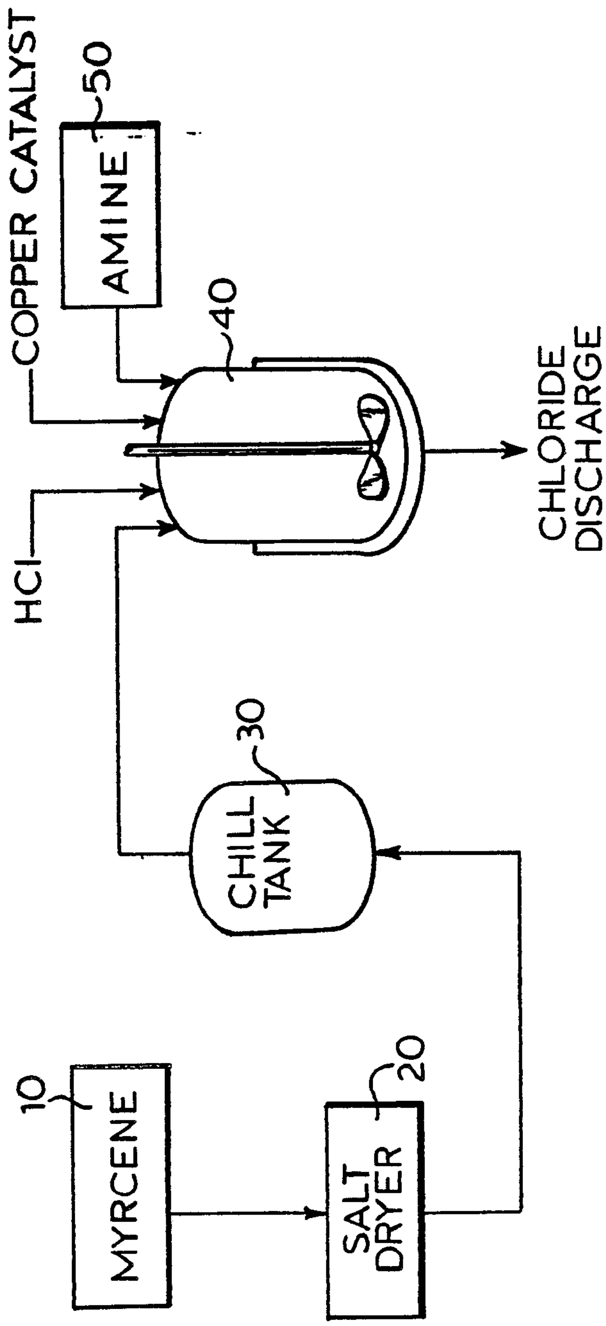
MYRCENE
10
SALT DRYER
20
CHILL TANK
30
HCl
COPPER CATALYST
AMINE
50
40
CHLORIDE DISCHARGE